# EUROPEAN PATENT SPECIFICATION

(11) **EP 2 766 011 B1**
(45) Date of publication and mention of the grant of the patent: **27.09.2017**
(21) Application number: 12780610.7
(22) Date of filing: 12.10.2012
(51) Int. Cl.: A61K 31/198, A61K 31/205, A61K 31/4439, A61K 31/519, A61K 31/5377, A61K 45/06, A61P 1/14, A61P 35/00

(54) **TREATMENT OF PHARMACOLOGICAL-INDUCED HYPOCHLORHYDRIA IN CANCER PATIENTS**
BEHANDLUNG VON PHARMAKOLOGISCH INDUZIERTER-HYPOCHLORHYDRIE IN KREBS-PATIENTEN
TRAITEMENT DE L'HYPOCHLORHYDRIE INDUITE PHARMACOLOGIQUEMENT CHEZ DES PATIENTS CANCÉREUX

(30) Priority: 13.10.2011 US 201161546814 P
(43) Date of publication of application: 20.08.2014
(73) Proprietor: Genentech, Inc., South San Francisco, CA 94080 (US); The Regents of the University of California, Oakland, CA 94607 (US)
(72) Inventor: BENET, Leslie Z., Oakland, California 94607 (US); DALZIEL, Gena, South San Francisco, California 94080 (US); DEAN, Brian, South San Francisco, California 94080 (US); DRESSER, Mark, South San Francisco, California 94080 (US); FRYMOYER, Adam, Palo Alto, California 94304 (US); HOLDEN, Scott, South San Francisco, California 94080 (US); JIN, Jin, South San Francisco, California 94080 (US); WARE, Joseph A., South San Francisco, California 94080 (US)
(74) Representative: Kiddle, Simon John
(86) International application number: PCT/US2012/059875
(87) International publication number: WO 2013/055996

(56) References cited:
- RAYNAUD F I ET AL: "Biological properties of potent inhibitors of class I phosphatidylinositide 3-kinases: From PI-103 through PI-540, PI-620 to the oral agent GDC-0941", MOLECULAR CANCER THERAPEUTICS 20090701 AMERICAN ASSOCIATION FOR CANCER RESEARCH INC. USA, vol. 8, no. 7, 1 July 2009 (2009-07-01), pages 1725-1738, XP002687849, ISSN: 1535-7163
- KANG BAOLIN ET AL: "Effect of omeprazole-induced achlorhydria on trefoil peptide expression in the rat stomach", JOURNAL OF GASTROENTEROLOGY AND HEPATOLOGY, WILEY INTERSCIENCE, MELBOURNE, AU, vol. 16, no. 11, 1 November 2001 (2001-11-01), pages 1222-1227, XP002313929, ISSN: 0815-9319, DOI: 10.1046/J.1440-1746.2001.02609.X
- KNAPP M J ET AL: "Modification of gastric pH with oral glutamic acid hydrochloride", CLINICAL PHARMACY 1991 US, vol. 10, no. 11, 1991, pages 866-869, XP009165029, ISSN: 0278-2677
- RUSSELL T L ET AL: "pH-related changes in the absorption of dipyridamole in the elderly.", PHARMACEUTICAL RESEARCH JAN 1994, vol. 11, no. 1, January 1994 (1994-01), pages 136-143, XP002687850, ISSN: 0724-8741
- ADRIAN J FOLKES ET AL: "The identification of 2-81H-Indazol-4-yl)-6-(4-methanesulfonyl-p iperazin-1-ylmethyl)-4-morpholin-4-yl-thie no[3,2-d]pyrimidine (GDC-0941) as a Potent, Selective, Orally Bioavailable Inhibitor of Class I PI3 Kinase for the Treatment of Cancer", JOURNAL OF MEDICINAL CHEMISTRY, AMERICAN CHEMICAL SOCIETY, US, vol. 51, no. 18, 1 January 2008 (2008-01-01), pages 5522-5532, XP002670417, ISSN: 0022-2623, DOI: 10.1021/JM800295D [retrieved on 2008-08-29]
- MONOBE MANAMI ET AL: "Glycine betaine, a beer component, protects radiation-induced injury.", JOURNAL OF RADIATION RESEARCH MAR 2005, vol. 46, no. 1, March 2005 (2005-03), pages 117-121, XP002687851, ISSN: 0449-3060
- T. L. JORGENSEN; J. HALLAS; J. HERRSTEDT: "Polypharmacy in elderly cancer patients", AMERICAN SOCIETY OF CLINICAL ONCOLOGY, 2010, XP28452665, cited in the application
- Joseph Ware: "Drug absorption interactgions between molecular targeted oncology agents and acid reducing agents", Genentech , 3 November 2014 (2014-11-03), Retrieved from the Internet: URL:https://zerista.s3.amazonaws.com/item_ files/1894/attachments/31490/original/335. pdf [retrieved on 2017-03-29]
- PANG JODIE ET AL: "Pharmacokinetics and absorption of the anticancer agents dasatinib and GDC-0941 under various gastric conditions in dogs--reversing the effect of elevated gastric pH with betaine HCl.", MOLECULAR PHARMACEUTICS 04 NOV 2013, vol. 10, no. 11, 4 November 2013 (2013-11-04), pages 4024-4031, ISSN: 1543-8392

## Description

### FIELD OF THE INVENTION

The present invention relates to methods using a PI3K inhibitor compound, GDC-0941, for the treatment of cancer.

### BACKGROUND

The stomach is a large organ that can be divided into 3 main zones that are involved in the digestion of foodstuff and the sterilization of liquids and water. The functional process of the stomach has been commonly divided into two zones: Upper Stomach, and Lower Stomach. The upper stomach, composed of the fundus and upper body, shows low frequency, sustained contractions that are responsible for generating a basal pressure within the stomach. These tonic contractions also generate a pressure gradient from the stomach to the small intestine and are responsible for gastric emptying. Swallowing food and the consequent gastric distention that occurs acts to inhibit contraction of this region, allowing the stomach to balloon out forming a large reservoir without a significant increase in pressure. The lower stomach is involved in the grinding and liquefaction of the foodstuffs by the secretion of HCl from the parietal cells found in this section of the stomach.

Generation of concentrated 0.16N hydrochloric acid by the mammalian parietal cell involves a complex combination of neuronal and hormonal regulatory feedback loops (Hersey S J, Sachs G. Gastric acid secretion. Physiol Rev 1995; 75:155-189; Sachs G, Prinz C, Loo D, Bamberg K, Besancon M, Shin J M. Gastric acid secretion: activation and inhibition. Yale J Biol Med 1994; 67:81-95; Sachs G. Physiology of the parietal cell and therapeutic implications. Pharmacotherapy 2003; 23:68 S-73S). Following activation of the cell there is a complex cellular transfer of ions that allows for the formation of acid (Horie S, Yano S, Watanabe K. Effects of drugs acting on Cl(-)-. Eur J Pharmacol 1992; 229:15-19; Helander H F, Keeling D J. Cell biology of gastric acid secretion. Baillieres Clin Gastroenterol 1993; 7:1-21; Soumarmon A, Lewin M J. Gastric (H+,K+)-ATPase. Biochimie 1986; 68:1287-1291; Wolfe M M, Welage L S, Sachs G. Proton pump inhibitors and gastric acid secretion. Am J Gastroenterol 2001; 96:3467-3468). A disruption in any of these components (secretory receptors, or ion transporters) can lead to either a cessation in the secretion of acid, or in the hypersecretion of acid. In the latter over 30 million patients per year suffer from symptoms of acid related diseases with the numbers increasing annually (Aihara T, Nakamura E, Amagase K, Tomita K, Fujishita T, Furutani K, Okabe S. Pharmacological control of gastric acid secretion for the treatment of acid-related peptic disease: past, present, and future. Pharmacol Ther 2003; 98:109-127; Gardner J D, Sloan S, Miner P B, Robinson M. Meal-stimulated gastric acid secretion and integrated gastric acidity in gastro-oesophageal reflux disease. Aliment Pharmacol Ther (2003) 17:945-953; Williams J L. Gastroesophageal reflux disease: clinical manifestations. Gastroenterol Nurs (2003) 26:195-200; Lehmann F, Hildebrand P, Beglinger C. New molecular targets for treatment of peptic ulcer disease. Drugs (2003) 63:1785-1797). Clinically the uncontrolled release or the continued hypersecretion of acid can lead to changes in both gastric and intestinal epithelium, but can in more serious cases lead to erosions of the esophagus that can result in metaplasia and death (Brzozowski T, Konturek P C, Konturek S J, Drozdowicz D, Kwiecien S, Pajdo R, Bielanski W, Hahn E G. "Role of gastric acid secretion in progression of acute gastric erosions induced by ischemia-reperfusion into gastric ulcers." Eur J Pharmacol 2000; 398:147-158; Franzin G, Manfrini C, Musola R, Rodella S, Fratton A. "Chronic erosions of the stomach--a clinical, endoscopic and histological evaluation." Endoscopy 1984; 16:1-5; Raugstad T S, Svanes K, Ulven A, Molster A. "Interaction between acute gastric ulcer and epinephrine-induced mucosal erosions in the rat: the significance of gastric acid secretion." Digestion 1979; 19:70-72). In an attempt to design therapies to prevent hyperacid secretion a variety of approaches have been employed in recent years with two of the most successful being: a) inhibition of the Histamine receptor on the basolateral membrane of the parietal cell, b) proton pump specific drugs targeted against the H⁺/K⁺-ATPase, the so called proton pump inhibitors, "PPI" (Bell N J, Hunt R H. Progress with proton pump inhibition. Yale J Biol Med 1992; 65:649-657; Garnett W R. Lansoprazole: a proton pump inhibitor. Ann Pharmacother 1996; 30:1425-1436; Robinson M. Drugs, bugs, and esophageal pH profiles. Yale J Biol Med 1999; 72:169-172). Both of these therapies have greatly improved the quality of life for patients suffering from this disease, however there is an ever increasing number of patients that have experienced recurrent disease while still taking the drugs (Tutuian R, Katz P O, Castell D O. "Nocturnal acid breakthrough: pH, drugs and bugs." Eur J Gastroenterol Hepatol 2004; 16:441-443; Adachi K, Komazawa Y, Fujishiro H, Mihara T, Ono M, Yuki M, Kawamura A, Karim Rumi M A, Amano Y, Kinoshita Y. "Nocturnal gastric acid breakthrough during the administration of rabeprazole and ranitidine in Helicobacter pylori-negative subjects: effects of different regimens." J Gastroenterol 2003; 38:830-835). Despite their high degree of efficacy and worldwide clinical use, failure in the treatment of acid related diseases has been reported and the degree and speed of onset of symptom relief are important to patients (Kleinman L, McIntosh E, Ryan M, Schmier J, Crawley J, Locke G R, III, De L G. Willingness to pay for complete symptom relief of gastroesophageal reflux disease. Arch Intern Med 2002; 162:1361-1366). It has been estimated that about 30% of GERD patients remain symptomatic on standard dose of PPI (Carlsson R, Galmiche J P, Dent J, Lundell L, Frison L. Prognostic factors influencing relapse of oesophagitis during maintenance therapy with antisecretory drugs: a meta-analysis of long-term omeprazole trials. Aliment Pharmacol Ther 1997; 11:473-482). Therapeutic oral doses of PPI reach steady state and thus achieve their maximal effective level after 4-5 days with typical dosing regimens (Tytgat G N. Shortcomings of the first-generation proton pump inhibitors. Eur J Gastroenterol Hepatol 2001; 13 Suppl 1:529-S33). This slow and cumulative onset of effect with PPI relates to their ability to inhibit only those pumps which are active when the PPI drug is available. After PPI administration, there is a return of acid secretion that is partly due to de novo synthesis of the enzyme (Gedda K, Scott D, Besancon M, Lorentzon P, Sachs G. "Turnover of the gastric H+,K(+)-adenosine triphosphatase alpha subunit and its effect on inhibition of rat gastric acid secretion." Gastroenterology 1995; 109:1134-1141).

Gastric acid aids protein digestion; facilitates the absorption of iron, calcium, and vitamin B12; and prevents bacterial overgrowth. When levels of acid and proteolytic enzymes overwhelm the mucosal defense mechanisms, ulcers occur. To avoid damage that is associated with these harsh conditions, gastric acid must be finely regulated by overlapping neural (e.g. acetylcholine), hormonal (e.g. gastrin and ghrelin), and paracrine (e.g. histamine and somatostatin) pathways, and more recently via the Calcium Sensing Receptor. Any long term alterations in any of these regulatory pathways leads to cell and tissue destruction and clinical manifestations such as peptic ulcer diseases, or gastroesophageal reflux disease (GERD). Two methods are commonly employed to treat the overproduction of acid: a) surgically, by elimination of the neuronal element (vagotomy) or b) pharmacologically, either through histamine 2 receptor antagonists (H2RA) or proton pump inhibitors (PPI) or a combination of both.

Proton pump inhibitors (PPI) such as omeprazole (PRILOSEC®, AstraZeneca) irreversibly inhibit gastric H⁺/K⁺-ATPase. Omeprazole, rabeprazole (ACIPHEX®, Janssen-Cilag), and lansoprazole (PREVACID®, Novartis) bind to multiple cysteine residues on the exofacial or luminal surface of the H⁺/K⁺-ATPase, and are activated in the acidic lumen of the gastric gland. In the resting cell the acid secreting pumps are internalized in a system of tubular vesicles, and are in such a conformational state that the PPI can only inhibit the H⁺/K⁺-ATPases which have already been activated and transferred to the apical surface of the parietal cell. PPI reduce gastric acid secretion, including acid secretion in the fundus (by inhibiting vacuolar H⁺-ATPase or H⁺/K⁺-ATPase) and upper body region of the stomach (by inhibiting H⁺/K⁺-ATPase), thus raising the pH of the stomach during resting phase as well as decreasing the duration of stomach acid release during a secretagogue phase. PPI are useful for treating conditions including dyspepsia, gastroesophogeal reflux disease (GERD), non-erosive reflux disease (NERD), Zollinger-Ellison syndrome (ZE disease), ulcer disease, and gastric cancer, as well as preventing or reducing the likelihood of ulcer disease (WO 2009/017624). In general, proton pump inhibitors are well tolerated, and the incidence of short-term adverse effects is relatively uncommon. The drastic change in pH and gastric acid levels in the gut caused by proton pump inhibitors may affect the bioavailability and absorption of orally administered therapeutics. Because the body uses gastric acid to release B₁₂ from food particles, decreased vitamin B₁₂ absorption may occur with long-term use of proton-pump inhibitors and may lead to Vitamin B12 deficiency. PPI, including esomeprazole (NEXIUM®, AstraZeneca), lansoprazole, omeprazole, dexlansoprazole, pantoprazole, and rabeprazole, are the most commonly prescribed medications in North America and Western Europe and are the optimal medications for the palliative relief of symptoms arising from gastroesophageal reflux disease (GERD) occurring in patients with both erosive and non-erosive esophagitis, treatment and prevention of peptic ulcer disease, as well as in many patients with nonulcer dyspepsia (Targownik, L. E., C. Metge, et al. (2007). "The prevalence of and the clinical and demographic characteristics associated with high-intensity proton pump inhibitor use." Am J Gastroenterol 102(5): 942-50). PPI are the most widely used gastric acid-reducing therapeutics because of their demonstrated overall safety, and have essentially replaced histamine H2-receptor antagonists (H2RAs) and other acid reducing agents for treatment of most chronic indications because of their perceived advantages including their prolonged pharmacologic effect (Yang, Y. X., S. Hennessy, et al. (2007). "Chronic proton pump inhibitor therapy and the risk of colorectal cancer." Gastroenterology 133(3): 748-54).

Many oncology patients use gastric acid-reducing therapeutics such as a PPI, an H2-receptor antagonist, or an antacid for gastric disturbances. H2-receptor antagonists (H2-RA) include cimetidine (TAGAMET®, GlaxoSmithKline), famotidine (PEPCID®, Johnson & Johnson/Merck), nizatidine (TAZAC®, AXID® (Eli Lilly), and ranitidine (ZANTAC®, Boehringer-Ingelheim). Antacids typically include aluminum hydroxide/carbonate, calcium hydroxide/carbonate, bismuth subsalicylate, or other buffering salts in their formulations. Most cancer patients participating in clinical trials take between 6-14 prescription drugs in addition to the test candidate (T. L. Jorgensen, J. Hallas, and J. Herrstedt. "Polypharmacy in elderly cancer patients", American Society of Clinical Oncology (2010) Chicago, IL: Journal of Clinical Oncology). In particular, many patients at some point receive some form of a gastric acid-reducing medication to treat gastrointestinal side effects, such as gastroesophageal reflux disease (GERD), dyspepsia or gastritis, frequently associated with their anti-cancer therapy. The bioavailability of certain orally administered therapeutics is influenced by pH in the gut. Solubility and permeability are important determinants of pharmacokinetics (PK) and drug absorption (Amidon et al (1995) Pharm. Res. 12:413; Wu and Benet (2005) Pharm. Res. 22:11), along with food intake, achlorhydria, and GI surgical resection. One of the largest contributors of intra and inter-subject pharmacokinetic variability occurs during the process of drug absorption. The estimated inter-subject variability in absorption K_{abs} rate ranges from 40 to >100% with inter-occasion variability (IOV) sometimes ranging from 40-60% (Sparreboom, A. and J. Verweij, Advances in cancer therapeutics. Clin Pharmacol Ther, 2009. 85(2): p. 113-7; Undevia, S.D., G. Gomez-Abuin, and M.J. Ratain, Pharmacokinetic variability of anticancer agents. Nat Rev Cancer, 2005. 5(6): p. 447-58). First pass metabolism and drug transport likely contributes to this variability in drug absorption (Wienkers, L.C. and T.G. Heath, "Predicting in vivo drug interactions from in vitro drug discovery data", Nat Rev Drug Discov, 2005. 4(10): p. 825-33; Giacomini, K.M., et al., "Membrane transporters in drug development", Nat Rev Drug Discov. 9(3): p. 215-36).

The intended pharmacologic effect of gastric acid-reducing agents on increasing the gastric pH can have significant impact on drug dissolution, absorption, and pharmacokinetics of orally administered cancer therapeutics with pH-dependent solubility (Duong, S. and M. Leung "Should the concomitant use of erlotinib and acid-reducing agents be avoided? The drug interaction between erlotinib and acid-reducing agents", J Oncol Pharm Pract; Eley, T., F. R. Luo, et al. (2009). "Phase I study of the effect of gastric acid pH modulators on the bioavailability of oral dasatinib in healthy subjects", J Clin Pharmacol 49(6): 700-9; Bergman et al (2007) "Pharmacokinetics of gefitinib in humans: the influence of gastrointestinal factors", Intl. J. Pharm. 341:134-142; Sparano et al (2009) "Effect of antacid on imatinib absorption", Cancer Chemother. Pharmacol. 63:525-528). A decrease in the overall exposure of an orally administered cancer therapeutic may lead to compromised pharmacodynamic effect and/or long-term multidrug resistance thereby impacting patient outcomes.

The majority (approximately 70%) of approved orally administered small molecule anti-cancer drugs exhibit pH-dependent solubility. Consequently, the oral bioavailability of these drugs may be significantly influenced by limited uptake and absorption in the gut at higher pH when co-administered with acid-reducing agents. These pH-dependent effects on exposure are most prominent for drugs that exhibit exponentially decreasing pH-solubility and when the maximum dose strength is not soluble in 250 mL of water at higher pH (above gastric pH 1-2). For example, increased gastric pH showed significantly decreased exposures of dasatinib (SPRYCEL®, Bristol-Myers), gefitinib, erlotinib, and nilotinib (Budha NR, Frymoyer A, Smelick GS, et al. (2012) Drug absorption interactions between oral targeted anticancer agents and PPIs: Is pH-dependent solubility the Achilles heel of targeted therapy? Clinical pharmacology and therapeutics; 92:203-213). Although the potential impact of concomitant gastric acid-reducing therapy on the efficacy of these approved drugs has not been clearly defined, it is plausible to hypothesize that altered drug solubility and subsequent decreases in drug exposure may be a contributing factor to the development of acquired drug-resistance.

Hypochlorhydria, or achlorhydria, is a condition characterized by abnormally low levels of hydrochloric acid in the stomach is most commonly a result of gastric atrophy. Gastric atrophy can be caused from various pathophysiologic states most common of which is acute gastritis initiated by infection with Helicobacter pylori. Hypo- or achlorhydria is also more commonly seen with advanced age. Less common causes include antrectomy with vagotomy for peptic ulcer disease and subtotal gastrectomy for cancer.

Predictable oral delivery of targeted kinase pathway inhibitors remains an unmet medical need. Most cancer patients are on a number of drugs which alter gastric pH, stomach emptying, and/or GI motility. Approximately 10% of the adult US population is on a PPI and it is estimated by oncologists that 20-100% of cancer patients may take PPI to manage palliative symptoms associated with gastroesophageal reflux disease (GERD). The prevalence of acid-reducing agents is in various cancer populations has been determined using an epidemiological approach. Two large, representative healthcare databases were employed: a retrospective cross-sectional analysis using the MarketScan (N=1,776,443) and Veterans Affairs (VA, N=1,171,833) databases. Cancer was defined as ≥ 2 ICD-9-CM CA-specific codes within 180 days of each other and ≥ 1 day apart. Patient records between Oct 1999-Jan 2011 and Jan 1999-Jun 2009 were examined for the VA and MarketScan Databases, respectively. Prescriptions and refills of acid-reducing agents (ARAs) were identified from 1 month prior to 6 months after the index date of cancer. The total prevalence of acid reducing agents in cancer patients was 20% and 33% for the MarketScan and VA databases, respectively. Increased gastric pH may have a profound impact on drug solubility/dissolution and permeability thereby impacting drug absorption and predictable systemic exposure and patient response. Re-acidification of the gastric lumen after PPI treatment may temporarily overcome pH-mediated drug-drug interactions (DDI) and enhance exposure (AUC/Cmax) and lead to desired therapeutic outcome.

### SUMMARY

Described herein are methods of treating pharmacological-induced hypochlorhydria in cancer patients with a re-acidification compound. These methods encompass modes of administration, dosing, scheduling, and therapeutic regimens.

The present invention provides a re-acidification compound and GDC-0941 for use in a method of treating a hyperproliferative disorder as defined in claim 1. Specifically, the present invention provides a re-acidification compound and GDC-0941 having the formula: 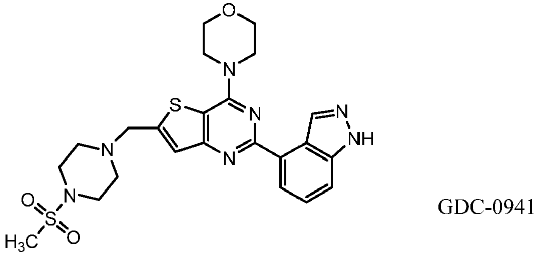 for use in a method of treating a hyperproliferative disorder, the method comprising administering a re-acidification compound and GDC-0941 to a patient receiving a gastric acid-reducing therapeutic selected from a proton-pump inhibitor, an H2-receptor antagonist, and an antacid, wherein the re-acidification compound is selected from betaine hydrochloride and glutamic acid hydrochloride.

Cancer patients being treated with PI3K inhibitor GDC-0941, 4-(2-(1H-indazol-4-yl)-6-((4-(methylsulfonyl)piperazin-1-yl)methyl)thieno[3,2-d]pyrimidin-4-yl)morpholine (CAS Reg. No. 957054-30-7), who are also on a gastric acid-reducing therapeutic such as a proton-pump inhibitor, an H2-receptor antagonist, or an antacid, are further treated with a re-acidification compound to improve the bioavailability of GDC-0941 by increasing gastric acidity and decreasing the pH in the stomach.

Methods described herein include wherein the structure of the PK/PD model describing the effects of a gastric acid-reducing therapeutic on the pharmacokinetics of GDC-0941 is established based on clinical data. Methods include wherein the PK/PD simulation in combination with a biomarker of gastric acid production, using the established PK/PD model can be used to predict effects of a gastric acid-reducing therapeutic with acid re-acidification on the pharmacokinetics of GDC-0941, optimize clinical trial design, provide individual patient dosing recommendation, and predict substrate PK profile in patients with varying gastric pH (such as GERD).

### BRIEF DESCRIPTION OF THE FIGURES

Figure 1 shows the Therapeutic Window and Anticipated Outcome of PPI-Targeted Agent Interaction.
Figure 2 shows the pH-dependent solubility curve at 37 °C of GDC-0941; 4-(2-(1H-indazol-4-yl)-6-((4-(methylsulfonyl)piperazin-1-yl)methyl)thieno[3,2-d]pyrimidin-4-yl)morpholino.
Figure 3 shows improved in vitro dissolution of GDC-0941 in the presence of betaine-HCl.
Figure 4 shows re-acidification by betaine-HCl in dogs improves the pharmacokinetics of GDC-0941.
Figure 5 shows the effects on bioavailability of dasatinib (Figure 5A and 5C) and gastric pH (Figure 5B) in dogs pre-treated with famotidine or pentagastrin, and the effects of betaine-HCl.
Figure 6 shows rabeprazole (PPI) significantly reduces GDC-0941 AUC and Cmax in the fasted or fed state in healthy volunteers.
Figure 7 shows rabeprazole (PPI) significantly reduces GDC-0941 AUC in phase I cancer patients
Figure 8 shows improved dasatinib exposure (AUC) after re-acidification by betaine-HCl in a healthy volunteer with rabeprazole-induced hypochlorhydria with pH monitored by a Heidelberg capsule.
Figure 9 shows reversal of pharmacological-induced hypochlorhydria after re-acidification by betaine HCl (BHCl) in healthy volunteers with rabeprazole-induced hypochlorhydria.
Figure 10 shows the Quantitative Pharmacological Model for the pharmacokinetics/pharmacodynamics (PK/PD) of GDC-0941 and dasatinib.

### DETAILED DESCRIPTION OF EMBODIMENTS OF THE INVENTION

Unless defined otherwise, technical and scientific terms used herein have the same meaning as commonly understood by one of ordinary skill in the art to which this invention belongs, and are consistent with:

### DEFINITIONS

The words "comprise," "comprising," "include," "including," and "includes" when used in this specification and claims are intended to specify the presence of stated features, integers, components, or steps, but they do not preclude the presence or addition of one or more other features, integers, components, steps, or groups thereof.

The term "re-acidification compound" refers to a compound administered to a patient on gastric acid-reducing therapy during treatment with a victim drug such as GDC-0941 which improves the bioavailability of the victim drug. Exemplary re-acidification compounds include, but are not limited to, betaine HCl, betaine citrate, and other betaine salts, and glutamic acid hydrochloride (ACIDULIN®, Eli Lilly), as well as other glutamate salts.

The terms "treat" and "treatment" refer to both therapeutic treatment and prophylactic or preventative measures, wherein the object is to prevent or slow down (lessen) an undesired physiological change or disorder, such as the growth, development or spread of a hyperproliferative condition, such as cancer. For purposes of this invention, beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder or those in which the condition or disorder is to be prevented.

The phrase "therapeutically effective amount" means an amount of a compound of the present invention that (i) treats the particular disease, condition, or disorder, (ii) attenuates, ameliorates, or eliminates one or more symptoms of the particular disease, condition, or disorder, or (iii) prevents or delays the onset of one or more symptoms of the particular disease, condition, or disorder described herein. In the case of cancer, the therapeutically effective amount of the drug may reduce the number of cancer cells; reduce the tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the cancer. To the extent the drug may prevent growth and/or kill existing cancer cells, it may be cytostatic and/or cytotoxic. For cancer therapy, efficacy can be measured, for example, by assessing the time to disease progression (TTP) and/or determining the response rate (RR).

The terms "cancer" and "cancerous" refer to or describe the physiological condition in mammals that is typically characterized by unregulated cell growth. A "tumor" comprises one or more cancerous cells. Examples of cancer include, but are not limited to, mesothelioma, carcinoma, lymphoma, blastoma, sarcoma, and leukemia or lymphoid malignancies. More particular examples of such cancers include squamous cell cancer (*e.g.,* epithelial squamous cell cancer), lung cancer including small- cell lung cancer, non-small cell lung cancer ("NSCLC"), adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, as well as head and neck cancer.

"Progression-Free Survival" (PFS) is the time from the first day of treatment to documented disease progression (including isolated CNS progression) or death from any cause related to the disease on study, whichever occurs first.

"Overall Survival" is the time from first day of treatment to death from any cause related to the disease.

"Dose-Limiting Toxicity" (DLT) is defined by a decrease in predicted diffusion capacity of carbon monoxide (DLco) of ≥ 20 percentage points (corrected for hemoglobin and alveolar volume).

The term "package insert" is used to refer to instructions customarily included in commercial packages of therapeutic products, that contain information about the indications, usage, dosage, administration, contraindications and/or warnings concerning the use of such therapeutic products.

An "adverse event" (AE) is any unfavorable and unintended sign, symptom, or disease temporally associated with the use of an investigational (medicinal) product or other protocol-imposed intervention, regardless of attribution; and includes: AEs not previously observed in the patient that emerge during the protocol-specified AE reporting period, including signs or symptoms associated with breast cancer that were not present before the AE reporting period; complications that occur as a result of protocol-mandated interventions (e.g., invasive procedures such as biopsies); if applicable, AE that occur before assignment of study treatment associated with medication washout, no treatment run-in, or other protocol-mandated intervention; Preexisting medical conditions (other than the condition being studied) judged by the investigator to have worsened in severity or frequency or changed in character during the protocol-specified AE reporting period

An adverse event is classified as a "Serious Adverse Events" (SAE) if it meets the following criteria: results in death (i.e., the AE actually causes or leads to death); life threatening (i.e., the AE, in the view of the investigator, places the patient at immediate risk of death, but not including an AE that, had it occurred in a more severe form, might have caused death); requires or prolongs inpatient hospitalization; results in persistent or significant disability/incapacity (i.e., the AE results in substantial disruption of the patient's ability to conduct normal life functions); results in a congenital anomaly/birth defect in a neonate/infant born to a mother exposed to the investigational product; or is considered a significant medical event by the investigator based on medical judgment (e.g., may jeopardize the patient or may require medical/surgical intervention to prevent one of the outcomes listed above). All AEs that do not meet any of the criteria for serious are regarded as non-serious AEs. The terms "severe" and "serious" are not synonymous. Severity (or intensity) refers to the grade of a specific AE, e.g., mild (Grade 1), moderate (Grade 2), or severe (Grade 3) myocardial infarction. "Serious" is a regulatory definition (see previous definition) and is based on patient or event outcome or action criteria usually associated with events that pose a threat to a patient's life or functioning. Seriousness (not severity) serves as the guide for defining regulatory reporting obligations from the Sponsor to applicable regulatory authorities. Severity and seriousness should be independently assessed when recording AEs and SAEs on the eCRF.

The terms "hypochlorhydria" and "achlorhydria" refer to states where the production of gastric acid in the stomach is low or absent, respectively. Gastric acid is a digestive fluid, formed in the stomach. It has a pH of about 1 to 2 and is composed of hydrochloric acid (HCl), about 0.5%, and potassium chloride (KCl) and sodium chloride (NaCl). Gastric acid plays a key role in digestion of proteins by activating digestive enzymes. Hypochlorhydria and achlorhydria are associated with various medical problems.

Betaine hydrochloride, also known as the hydrochloride salt of trimethylglycine, 2-trimethylammonioacetate, TMG, glycine betaine, betaine anhydrous, and N,N,N-trimethylglycine, has the formula: [(CH₃)₃N⁺CH₂CO₂H]⁺Cl⁻, (CAS Registry No. 107-43-7).

The Heidelberg pH Diagnostic System™ (Heidelberg Medical Incorporated, Mineral Bluff, GA) includes a tethered, micro-electronic capsule high-frequency transmitter encapsulated within a polyacrylate covering designed to be swallowed by the patient. The Heidelberg capsule is useful for measuring the pH levels in the digestive tract and can diagnose a patient who may have hypochlorhydria, hyperchlorhydria, achlorhydria, pyloric insufficiency, and heavy mucus. The Heidelberg pH Capsule is similar to the size of a vitamin capsule, measures 7.1 mm in diameter, and 15.4 mm in length, making it easy for the patient or test animal to swallow.

Dasatinib (SPRYCEL®, Bristol-Myers) is a small molecule kinase inhibitor indicated for the treatment of chronic myeloid leukemia. The anhydrous free base of dasatinib has a molecular weight of 488.01Da. Dasatinib is a free base with three ionization constants, 3.1, 6.8 and 10.8. Aqueous solubility of dasatinib is pH-dependent; the solubility decreases exponentially with increasing pH over the normal physiological range (Eley, T., et al., Phase I study of the effect of gastric acid pH modulators on the bioavailability of oral dasatinib in healthy subjects. J Clin Pharmacol, 2009. 49(6): p. 700-9). In this publication, healthy volunteers were dosed with oral dasatinib 50 mg BID and a single dose of famotidine (H2 receptor antagonist) 40 mg 10 hours prior to dasatinib. A 61 % reduction of bioavailability (AUC) was determined by measurement of plasma samples for dasatinib concentrations. Dasatinib solubility ranges from ∼18 mg/ml at pH 2.6, ∼ 690 µg/mL at pH 4.0, 205 µg/mL at pH 4.28, and to < 1 µg/ml at pH 7.0 at 24 °C. The highest commercial dose strength (140 mg) of dasatinib is not soluble in 250 mL of water in the pH range of 4.3 to 7.0 at 24 °C. Bioavailability of dasatinib is increased when administered with food. However, the increase in AUC_{inf} with a high fat meal (14%) and low fat meal (21 %) is well below the variability in AUC_{inf} in the fasted state, and therefore is not considered clinically relevant.

The variability in dasatinib exposure was previously examined using data from one phase I and five phase II studies (Dai, G., et al., "Importance of characterizing determinants of variability in exposure: application to dasatinib in subjects with chronic myeloid leukemia." J Clin Pharmacol, 2008. 48(11):1254-69). Between-subject variability in Ka exceeded 100% whereas the between-subject variability in relative bioavailability (F_{R}) was 33%. The inter-occasion variability (IOV) within a subject in F_{R} was estimated to be 44%. The use of PPI decreased the F_{R} of dasatinib by 17% (not statistically significant). Similarly, the antacids and H2-RAs did not show any significant effect on dasatinib F_{R}. The absence of effect of acid reducing agents on F_{R} could be due to the low number of subjects in the data (3 on antacids and 10 on H2RA), non-availability of actual dosing times for these agents, and multiple interacting medications (Dai, G., et al., "Importance of characterizing determinants of variability in exposure: application to dasatinib in subjects with chronic myeloid leukemia", J Clin Pharmacol, 2008. 48(11): p. 1254-69). Due to the exceedingly high variability in drug absorption, complex patient population and multiple interacting medications, it is difficult to assess the impact of a PPI on dasatinib absorption using uncontrolled population pharmacokinetic designs within a larger study. Using a well controlled cross-over clinical pharmacology study in healthy volunteers, the effect of acid suppressive therapy on dasatinib was evaluated in 22 healthy subjects. Administration of a single 50-mg dose of dasatinib 10 hours following 40 mg of famotidine reduced both the AUC₀₋₁₂ and Cₘₐₓ of dasatinib by ∼60% (Eley, T., et al., "Phase I study of the effect of gastric acid pH modulators on the bioavailability of oral dasatinib in healthy subjects", J Clin Pharmacol, 2009. 49(6): p. 700-9). In contrast, the exposure of dasatinib is unchanged when antacid containing aluminum/magnesium hydroxides (Maalox®, 30 mL) was administered 2 hours prior to dasatinib administration. However, the co-administration of dasatinib with Maalox® resulted in decreased exposure of dasatinib by 55 to 58%. In another study, the effect of a proton pump inhibitor, omeprazole was investigated on the pharmacokinetics of 100 mg of dasatinib in 14 healthy subjects. Dasatinib bioavailability was reduced by ∼40% after a 40 mg dose of omeprazole daily for 4 days. The AUC_{inf} and Cₘₐₓ of dasatinib were decreased by 43% and 42% respectively. As a result, the concomitant use of H₂ antagonists, antacids or proton pump inhibitors with dasatinib is not recommended and mentioned accordingly in the prescribing information.

### GDC-0941

GDC-0941 (Genentech Inc.), is a selective, orally bioavailable thienopyrimidine inhibitor of PI3K with promising pharmacokinetic and pharmaceutical properties (Folkes et al (2008) Jour. of Med. Chem. 51(18):5522-5532; Edgar et al (2010) Cancer Res. 70(3):1164-1172; Sutherlin et al (2010) Jour. of Med. Chem. 53(3):1086-1097; US 7781433; US 7750002; Belvin et al, American Association for Cancer Research Annual Meeting 2008, 99th:April 15, Abstract 4004; Folkes et al, American Association for Cancer Research Annual Meeting 2008, 99th:April 14, Abstract LB-146; Friedman et al, American Association for Cancer Research Annual Meeting 2008, 99th:April 14, Abstract LB-110). GDC-0941 shows synergistic activity in vitro and in vivo in combination with certain chemotherapeutic agents against solid tumor cell lines (Belvin et al, "Combinations Of Phosphoinositide 3-Kinase Inhibitor Compounds And Chemotherapeutic Agents, And Methods Of Use", US 8247397) and hematological malignancies (Friedman and Ebens "Combinations Of Phosphoinositide 3-Kinase Inhibitor Compounds And Chemotherapeutic Agents For The Treatment Of B-Cell Malignancies", US 2010/0233164), including combinations of MEK inhibitors (Belvin et al, "Combinations of a PI3K inhibitor and a MEK inhibitor for treatment of metastatic solid tumors", US 2011/0086837) and anti-HER2/HER3 antibodies (Yao et al (2009) Clin. Cancer Res. 15(12):4147-4156; Juntilla et al (2009) Cancer Cell 15(5):429-440) and anti-HER2 antibody drug conjugates (WO 2009/117277).

GDC-0941 is named as 4-(2-(1H-indazol-4-yl)-6-((4-(methylsulfonyl)piperazin-1-yl)methyl)thieno[3,2-d]pyrimidin-4-yl)morpholine (CAS Reg. No. 957054-30-7), and has the structure:

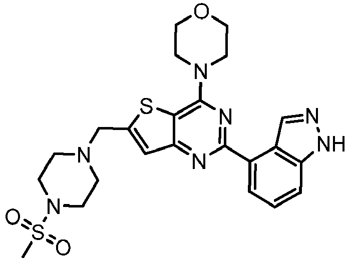

The anhydrous free base of GDC-0941 has a molecular weight of 513.64. The free base of GDC-0941 has two ionization constants 1.5 and 4.2. Formulated as the di-methanesulfonate salt (FW 705.85), GDC-0941 is undergoing clinical trials for the oral treatment of solid tumors, including breast cancer and non-small cell lung cancer (NSCLC). "A first-in-human phase I study to evaluate the pan-PI3K inhibitor GDC-0941 administered QD or BID in patients with advanced solid tumors", Wagner AJ, Von Hoff DH, LoRusso PM et al, American Society of Clinical Oncology Annual Meeting 2009, June 01 (Abs 3501); "A Phase Ib study to evaluate the pan-PI3K inhibitor GDC-0941 with paclitaxel and carboplatin with and without bevacizumab in non-small cell lung cancer patients", Soria JC, Gomez-Roca CA, Ware JA et al, EORTC-NCI-AACR International Congress 2010, 22nd:November 18 (Abs 421); "A phase Ib study to evaluate the PI3-kinase inhibitor GDC-0941 with paclitaxel (P) and carboplatin (C), with and without bevacizumab (BEV), in patients with advanced non-small cell lung cancer (NSCLC)", Besse B, Soria J, Gomez-Roca C et al, American Society of Clinical Oncology Annual Meeting 2011, 47th:June 06 (Abs 3044).

GDC-0941 is rapidly absorbed in cancer patients (Tmax about 2 hrs, with dose-proportional increases in AUC and Cmax. GDC-0941 demonstrates a blood concentration half-life of about 12-28 hours with an accumulation index at steady-state of 1.2 to 2.0. The estimated PK (pharmacokinetics) target in the most sensitive PI3K mutant model of 6 uM hr AUC was achieved in most patients at 80 mg.

The effect of acid reducing agents on weakly basic drugs is influenced by many factors, and DDI (drug-drug interaction) studies intended to distill this effect require careful consideration in study design. For example, the length of pre-treatment needed for testing the maximum pH effect will be of different duration for H₂RAs versus PPI. And even within a specific class such as the PPI, the gastric pH changes can vary in terms of magnitude and time-course. Further, the relationship between dose of test victim drug (those with pH-dependent solubility) and potential effect is usually non-linear. Additionally, apart from the pH-mediated effect, antacids can have effects on absorption of drugs by non-specific 'drug' adsorption. All these factors need to be considered for interpreting the results from interaction studies and recommendations on how to treat cancer patients who possess GERD.

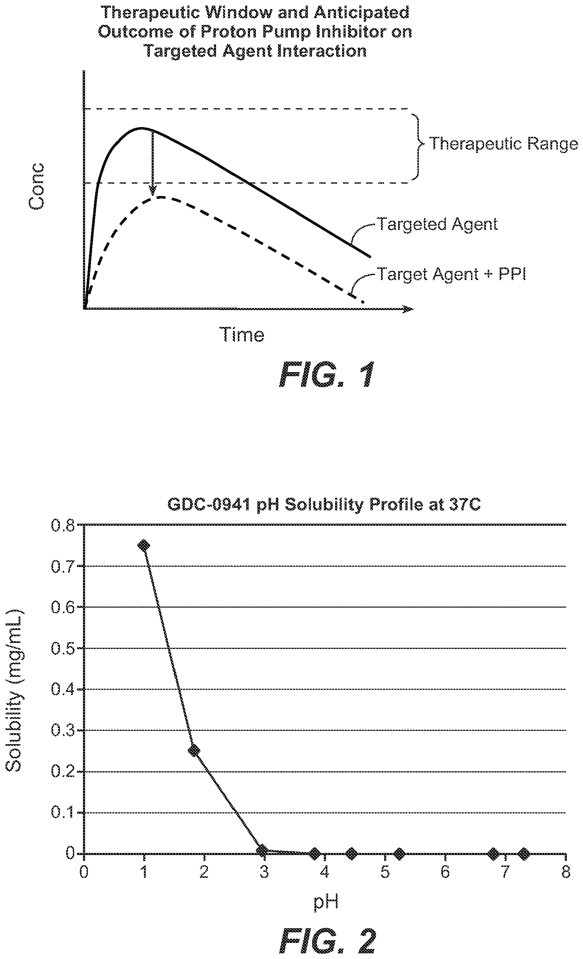

Figure 1 shows a hypothetical therapeutic range of plasma concentrations for a drug and the potential impact of PPI related drug interaction. The upper curve represents plasma exposures of a drug under the normal pH environment of the GI, while the lower curve depicts the impact of increased pH, due to a PPI, on a drug with poor solubility at higher pH and a reduced maximum absorbable dose.

### pH Solubility Profile for GDC-0941

Figure 2 depicts the pH-dependent solubility of GDC-0941. Considering that GDC-0941 is a weakly basic compound with pKa values of 1.5 and 4.2, it exhibits a pH-dependent solubility profile. The solubility data (shown in Figure 2) is measured after equilibrating for 48 hours at 37 °C. Moderate solubility of approximately 0.8 mg/mL to 8 µg/mL is observed at pH 1.0 through 3.0 respectively. As pH is increased, the solubility decreases. At all tested pH values above pH 4.4, the compound is practically insoluble (USP definition), with a measured solubility of approximately 1 µg/mL. This steep drop in solubility at high pH values suggests that absorption of GDC-0941 may be pH dependent. Therefore, sufficient gastric acidity maybe required for adequate in vivo dissolution and absorption. The steepest part of the curve is in the region which is impacted most by acid reducing agents such as PPI.

### Dissolution of GDC-0941 with a re-acidification compound

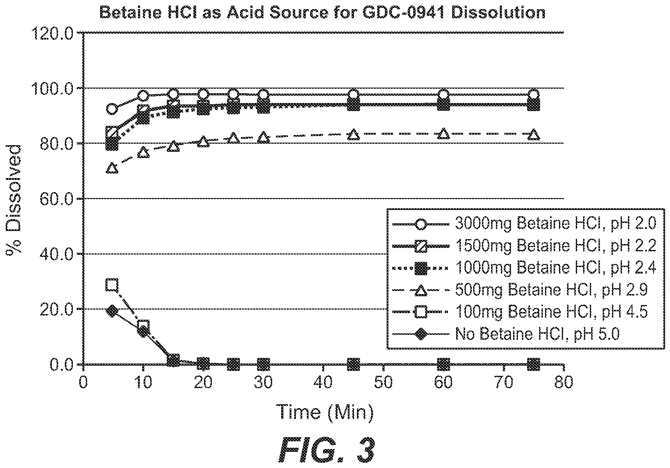

The pH-dependent release profile for GDC-0941 was evaluated in vitro using USP dissolution apparatus II. The dissolution test was performed on individual GDC-0941 tablets in 900 ml of 2mM sodium acetate buffer (pH 5.0) containing different quantities of betaine HCl (100 mg to 3000 mg). A control was run as well, in which no betaine HCl was added to the dissolution media. The low buffer capacity media and initial pH of 5.0 were selected to simulate achlorhydridic conditions. The dissolution data indicate that addition of the betaine HCl results in a decrease in media pH and a corresponding improvement in tablet dissolution rate (Figure 3). At pH 4.5 and pH 5.0 (achieved by addition of 100 mg and no betaine HCl), almost all of the drug that is released at early time points had precipitated out of solution. By 20 minutes, less than 1% of the active drug content remained in solution. Similar precipitation was not observed under conditions in which 500 mg or more betaine HCl was added to the media. In fact, greater than 90% of drug could be rapidly dissolved and maintained in solution if the media contained enough betaine HCl to lower the pH to 2.4 or below. This in vitro dissolution data demonstrates that betaine HCl can be used as a re-acidification agent to improve drug release and solubility of GDC-0941 (Example 4).

### Oral exposure of dasatinib in dogs with famotidine- and pentagastrin- or betaine HCl-induced hypochlorhydria and hyperchlorhydria, respectively

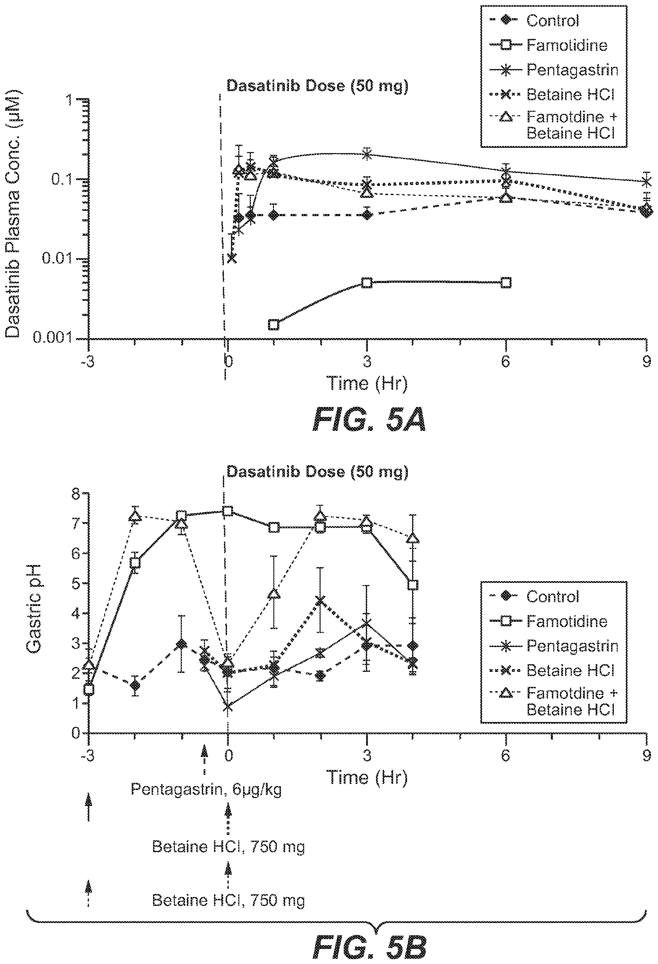

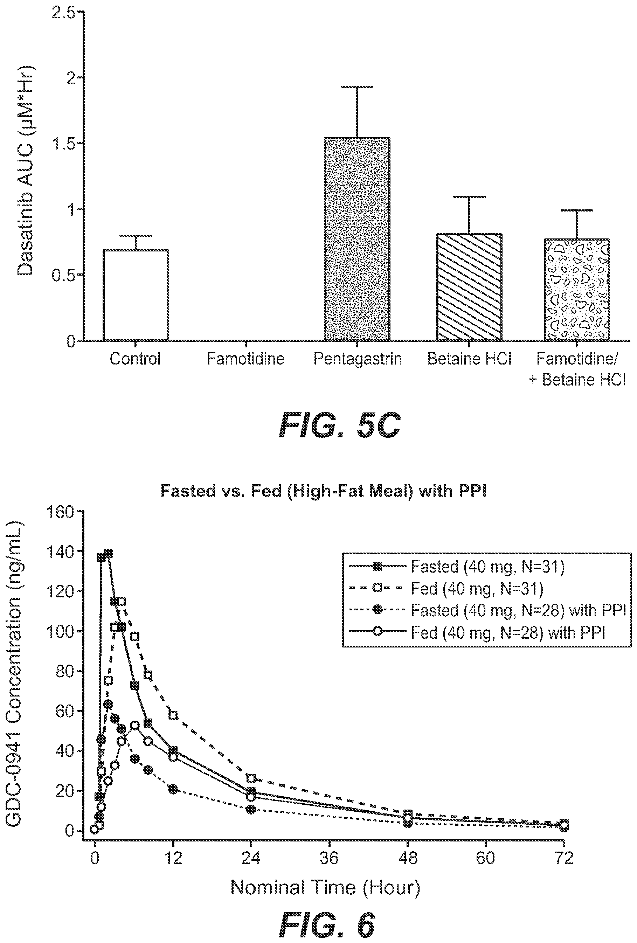

Dasatinib was studied as a model, surrogate for GDC-0941 due to its pH-dependent solubility (Figure 5). Dasatinib is thus likely to show similar dissolution effects as GDC-0941 in the stomach in response to re-acidification compounds.

Dogs were treated with dasatinib 50 mg tablet (Example 5). Dogs in each group received the following pretreatment: Group 1: no pretreatment (control); Group 2: famotidine 40 mg tablet orally, 3 hours prior to dasatinib; Group 3: pentagastrin by intramuscular injection (6 mg/kg) 30 min before dasatinib administration; Groups 4 and 5: Two betaine HCl 750 mg tablets were administered to the two groups as follows: one betaine HCl tablet orally 5 min before dasatinib and the other 20 min before dasatinib Group 5 was also treated with famotidine 40 mg tablet orally, 3 hours prior to dasatinib. Figure 5A shows dasatinib plasma concentration over time. Betaine HCl performs as well as pentagastrin, and improves dasatinib/famotidine exposure (AUC, Figure 5C).

In this experiment, pH was monitored (Figure 5B) and it was found that in dogs pretreated with the H2-receptor antagonist, famotidine, had significant hypochlorhydria (increased gastric pH). In the dogs treated with famotidine + betaine HCl, a reversal of hypochlorhydria was noted with gastric pH rapidly returning to normal acid levels. Dasatinib pharmacokinetics was significantly altered by changing gastric pH in both normal and hypochlorhydric state. In particular, dasatinib + famotidine without betaine HCl were found to have dasatinib levels at or below the limits of assay detection (Figure 5C).

### Oral exposure of GDC-0941 in dogs with famotidine- and pentagastrin- or betaine HCl-induced hypochlorhydria and hyperchlorhydria, respectively

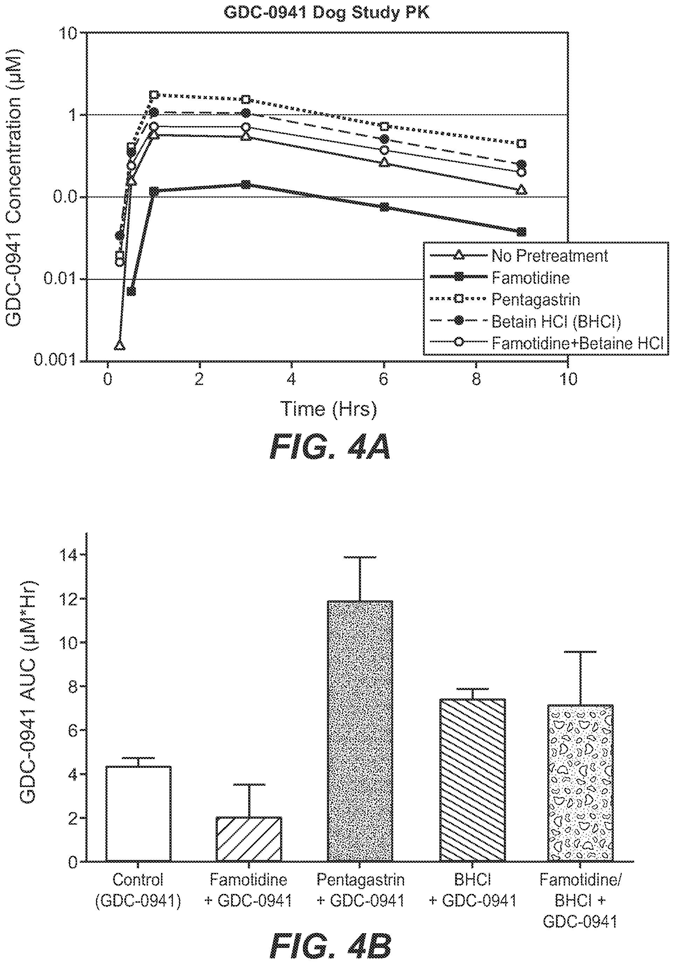

The oral exposure of GDC-0941 in dogs was measured by plasma concentration of GDC-0941 over time after initial dosing with a 40mg tablet (Figure 4), and as described in Example 6. Group 1 dogs received only GDC-0941, 2 x 20 mg tablets. Group 2 dogs were pretreated with famotidine 40 mg tablet to induce hypochlorhydria and as a negative control. Group 3 dogs were pretreated with pentagastrin by intramuscular injection 6 mg/kg. Pentagastrin is a synthetic peptide which stimulates the secretion of gastrin, pepsin, and intrinsic factor, and lowers the pH of the gut through physiologic stimulation of acid secretion. Group 4 dogs were treated with re-acidification compound, betaine HCl, 2 x 750 mg tablets administered simultaneously with GDC-0941, 2 x 20 mg tablets. Group 5 dogs were pretreated with famotidine, then treated with betaine HCl, 2 x 750 mg tablets.

Figure 4 shows that betaine HCl improves GDC-0941 plasma exposure in dogs with famotidine-induced achlorhydria. The order of mean exposure from high to low are: Group 3 (pentagastrin) > Group 4 (betaine HCl) > Group 5 (famotidine + betaine HCl) > Group 1 (no pretreatment) > Group 2 (famotidine). Inter-animal variability is low at low gastric pH, and becomes higher at higher gastric pH. Pharmacokinetic variability is lowest in pentagastrin treated group (Group 3), and then betaine HCl treated group (Group 4). Variability is high in famotidine treated group (Group 2), and is the highest in the untreated group (Group 1). In Group 5 (famotidine + betaine HCl), animal D501 has markedly lower exposure than the other 3 animals. The mean AUC and SD excluding this animal (n=3) (AUC=7.12 uM*hr, SD=2.46) are similar to the AUC and SD of betaine HCl only pretreatment (Group 4) (AUC=7.38 uM*hr, SD=2.61). In a surprising and unexpected discovery, betaine HCl improves exposure of GDC-0941 when compared with control. It counteracts the effect of famotidine to give a higher exposure and lower inter-animal variability. Pentagastrin pretreatment gives the highest exposure and lowest inter-animal pharmacokinetic variability in the 5 groups.

### Dosing and scheduling of GDC-0941 in healthy volunteers treated with proton-pump inhibitors and in phase I cancer patients

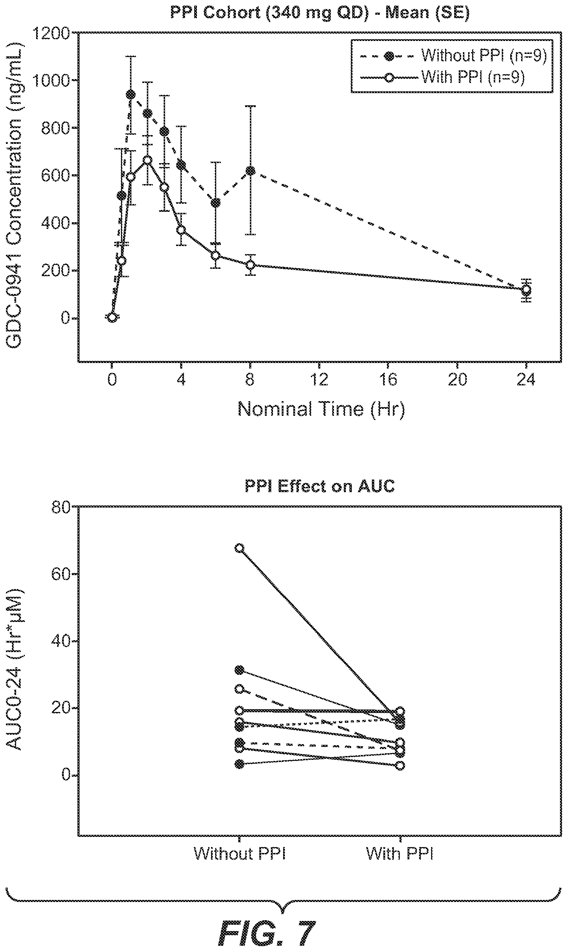

A clinical study was conducted to determine the effect of food and a proton pump inhibitor, rabeprazole, on the bioavailability and pharmacokinetics of the tablet formulation of GDC-0941 (Example 1). In the assessment of the effect of co-administration of a PPI with GDC-0941 on the pharmacokinetics of GDC-0941, rabeprazole 20 mg was administered for 5 consecutive days with co-administration of GDC-0941 40 mg prototype tablet (2 x 20 mg) on the fifth day either in the fasted state or following a high-fat meal. When comparing the effect of rabeprazole + GDC-0941 to GDC-0941 alone in the fasted state, GDC-0941 median Tₘₐₓ was approximately 2 hours (48.1 % CV). In the fasted state, both Cₘₐₓ and exposure to GDC-0941 decreased significantly (p < 0.001 for both parameters) in the presence of rabeprazole, with an estimated mean ratio (90% CI) of 0.31 (0.21, 0.46) and 0.46 (0.35, 0.61) for Cₘₐₓ and AUC_{0-∞}, respectively. When rabeprazole was administered in the presence of the high-fat meal, its affect on AUC_{0-∞} was slightly less dramatic (estimate of 0.60 with 90% CI of [0.50, 0.65], p < 0.001) and unchanged for the Cₘₐₓ (estimate of 0.43 with 90% CI of [0.37, 0.50], p < 0.001). No significant interaction between high-fat meal and rabeprazole was observed (p = 0.16 and p = 0.27 when testing for significance of the interaction in the two-way ANOVA model for Cₘₐₓ and AUC_{0-∞} respectively). Overall, the exposure to GDC-0941 during rabeprazole treatment, after administration of GDC-0941 in the presence of a high fat meal, did not overcome the decreased absorption of GDC-0941 caused by rabeprazole and it is hypothesized that the reacidification method proposed with betaine HCl will mitigate this decrease in GDC-0941 exposure caused by pharmacologic-induced hypochlorhydria. Figure 6 shows rabeprazole (PPI) significantly reduces the absorption of GDC-0941 in healthy volunteers in the fasted or fed state. Figure 7 shows the impact of rabeprazole on GDC-0941 absorption in Phase I cancer patients pretreated with PPI relative to control Day 1 pharmacokinetics.

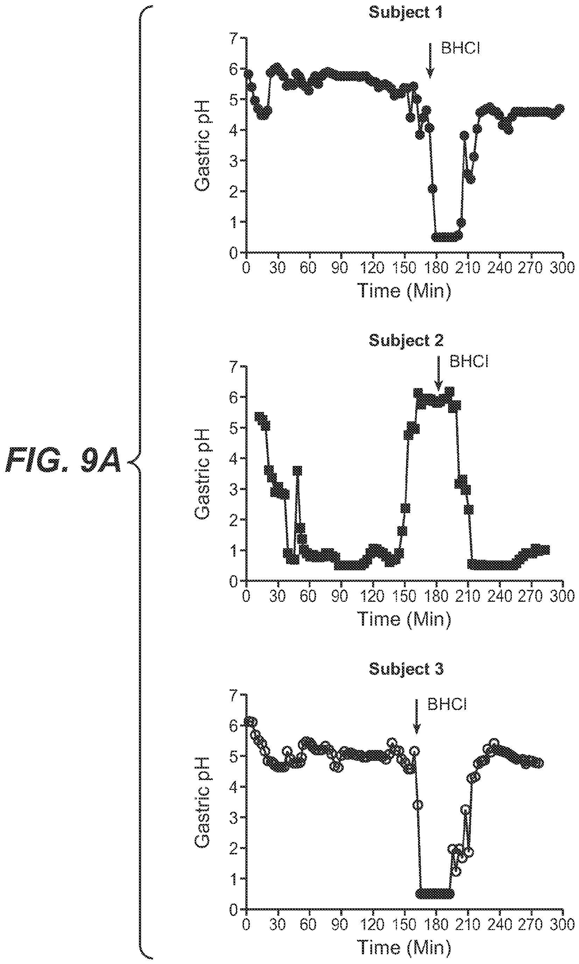

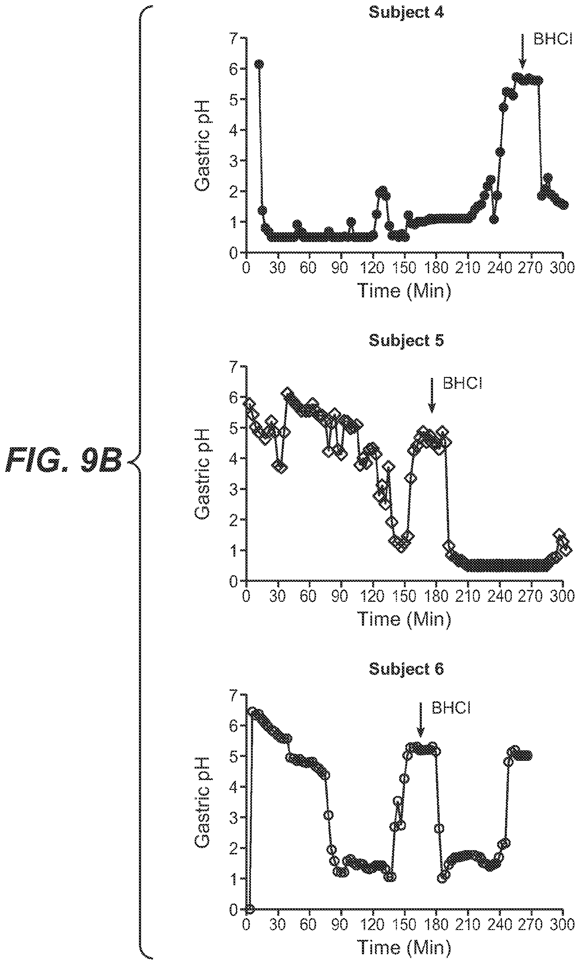

To clinically establish betaine hydrochloride's ability to re-acidify the stomach and the time course of this change, the gastric pH change after betaine hydrochloride was examined by administration in healthy volunteers with pharmacologically induced hypochlorhydria (rabeprazole). Figure 9 shows the re-acidification effect of betaine HCl administered to six healthy volunteers (Example 1) with rabeprazole-induced achlorhydria. The pH of the stomach was measured over time with a Heidelberg capsule with tether swallowed at t = 0. After establishing pharmacological-induced hypochlorhydria (pH>4), decrease in gastric pH was predictably measured in N=6 subjects after betaine HCl administration and allows sufficient acidic window for absorption of GDC-0941 and optimal pharmacokinetic profile. During the hypochlorhydria state induced by rabeprazole pretreatment, betaine HCl significantly (p<0.0001) lowered gastric pH by 4.54 (±0.46) units during the 30 min interval after dosing. Onset of effect of betaine HCl was rapid, with a mean time to pH <3 of 6.3 (±4.3) min, and its duration was adequate but temporary, with a gastric pH <3 and <4 lasting 72.5 (±32.9) and 76.8 (±30.4) min, respectively.

### Effects of Betaine HCl on dasatinib exposure during rabeprazole-induced hypochlorhydria in healthy volunteers

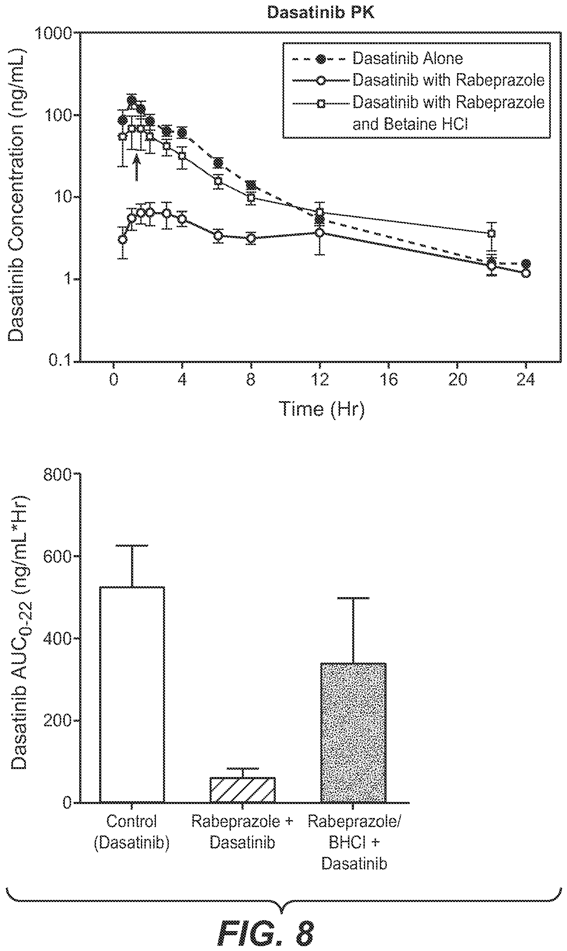

As shown in Figure 8 and Example 7, rabeprazole lowered dasatinib AUC 0-22 hr by 18-fold. However, compared with rabeprazole only, the co-administration of betaine HCl increased dasatinib AUC approximately 5-fold, thereby restoring DAS AUC to 80 ±32% of control. Therefore, betaine HCl (BHCl) improves dasatinib exposure during rabeprazole-induced hypochlorhydria. Dasatinib represents a surrogate molecule to GDC-0941 that displays pH-dependent solubility and is a BCS 2 classification. In the current U.S. prescribing information, physicians and patients are recommended not to co-administer H2-receptor antagonists (H2RA) or proton pump inhibitors (PPIs) because of this significant decrease in exposure. Based on the greatly improved pharmacokinetic profile of dasatinib during the hypochlorhydric state when betaine HCl is coadministered, it appears that exposure is restored to near control dasatinib levels.

### Effects of rabeprazole on the pharmacokinetics of GDC-0941 in patients with advanced solid tumors

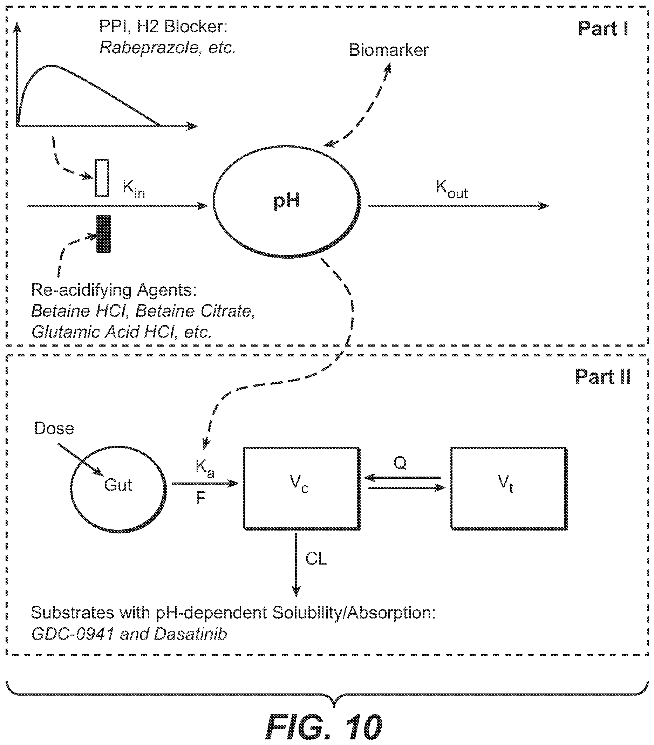

Pharmacokinetic/pharmacodynamic (PK/PD) modeling and simulation to describe and predict effects of pH-modifying agents on the pharmacokinetics of GDC-0941 PK/PD modeling and simulation has been widely used in current drug development to quantitative describe drug concentration (PK) and drug effect (PD). Figure 10 shows the schematic representation of the proposed model to describe and predict effects of pH-modifying agents on the pharmacokinetics of GDC-0941. Part I of the PK/PD model could quantitatively describe the effects of PPI, H2-receptor blocker, and/or re-acidifying agent on GI pH, as well as the potential relationship between biomarker and gastric pH, if applicable. Part II of the PK/PD model could describe the relationship between gastric pH and PK of substrates with pH-dependent solubility/absorption such as GDC-0941. Once established with clinical data, the PK/PD model could be used to predict effects of PPI, H2-receptor blocker, and/or re-acidifying agent on GI pH, and subsequent effects on PK of tested or untested substrates with pH-dependent solubility/absorption (such as GDC-0941) under various untested dosing scenarios. Simulations could be used to optimize clinical trial design and dosing recommendation. The model could also be used to predict substrate PK profile in patients with varying GI pH (such as GERD).

### EXAMPLES

### Example 1 Clinical study in healthy volunteers to determine the effect of food and a proton pump inhibitor (PPI) on the bioavailability and pharmacokinetics of the tablet formulation of GDC-0941

An open-label, randomized, two-part pharmacokinetic (PK) study in healthy volunteers was conducted to assess the relative bioavailability of capsule and tablet formulations of GDC-0941 in the fasted state (Part 1) and to determine the effect of food and a proton pump inhibitor (PPI) on the pharmacokinetics of the tablet formulation of GDC-0941 (Part 2). Healthy adult men and women aged 18-65 years were eligible for Part I and aged 18-45 years were eligible for Part 2. The upper age limit was reduced in Part 2 because decreased gastric acid production is observed more frequently in older adults. Eighteen subjects were enrolled in Part 1 and 31 subjects were enrolled in Part 2.

Part 1 of this study was a 2-period crossover design in which subjects were randomized to receive GDC-0941 60 mg in a fasting state either as tablets or capsules. GDC-0941 was rapidly absorbed with a median time to maximum concentration (Tₘₐₓ) of approximately 2 hours after administration for both formulations. The maximum plasma concentration (Cₘₐₓ) for GDC-0941 was not statistically different (p-value = 0.106) for the two formulations with an estimated mean ratio (tablet/capsule) of 0.86 (0.73, 1.00). The GDC-0941 tablet was absorbed at a similar rate (Tₘₐₓ/Cₘₐₓ) as the capsule.

The estimated geometric mean (± standard deviation) AUC_{0-∞} values for the GDC-0941 prototype tablet and capsules were 2450 ± 941 ng • h/mL and 2800 ± 882 ng • h/mL, respectively, with an estimated mean ratio (tablet/capsule) of 0.83 (0.73, 0.95). The extent of GDC-0941 exposure was slightly decreased relative to the capsule (p-value = 0.024; however, this slight decrease is not considered clinically significant and does not present a risk to patients of higher or significantly lower exposures than they would receive with the capsule formulation.

In the assessment of the effect of co-administration of a PPI with GDC-0941 on the pharmacokinetics of GDC-0941, rabeprazole 20 mg was administered for 5 consecutive days with co-administration of GDC-0941 40 mg (tablets) on the fifth day either in the fasted state or following a high-fat meal. When comparing the effect of GDC-0941 with rabeprazole to GDC-0941 without rabeprazole in the fasted state, GDC-0941 median Tₘₐₓ was approximately 2 hours. In the fasted state, both Cₘₐₓ and exposure to GDC-0941 decreased significantly (p < 0.001 for both parameters) in the presence of rabeprazole, with an estimated mean ratio (90% CI) of 0.31 (0.21, 0.46) and 0.46 (0.35, 0.61) for Cₘₐₓ and AUC_{0-∞}, respectively (Figure 6). The effect of the high-fat meal slightly attenuated the rabeprazole effect on exposure with an estimated mean ratio (90% CI) for GDC-0941 of 0.57 (0.50, 0.65) for AUC_{0-∞} and 0.43 (0.37, 0.50) for Cₘₐₓ. Using the known solubility and dissolution profile of GDC-0941 (Figures 2 and 3), pharmacokinetic profile of GDC-0941 in the dog (Figure 4), and the gastric pH-profile after betaine HCl (Figure 9) and the PK/PD model described in Figure 10, it is predicted that GDC-0941 exposure (AUC) can be increased during the hypochlorhdyric state induced by disease, H2-receptor antagonists (H2RA) or proton pump inhibitors (PPI).

### Example 2 Effects of rabeprazole on the pharmacokinetics of GDC-0941 in patients with advanced solid tumors

Approximately 100 patients with advanced solid tumors in Stage 2 were expected to be enrolled in a study with GDC-0941. The effect of a proton pump inhibitor (PPI) on the pharmacokinetics of GDC-0941 will be studied in up to 9 patients who are not participating in the PIK3CA mutation-positive cohort. Up to 9 patients in Stage 2 (excluding patients participating in the tumor PIK3CA mutation-positive cohort) will participate in an evaluation of the effect of a PPI on the pharmacokinetics of GDC-0941. Patients will be administered rabeprazole 20 mg (optimally in the morning) on Days 4 through 8 of Cycle 1. GDC-0941 will be co-administered with rabeprazole on Day 8 and blood samples will be collected for PK analyses. Treatment with GDC-0941 alone will continue starting on Day 9. Results of this study are shown in Figure 7. Overall, a 40% decrease of GDC-0941 was measured in subjects pretreated with rabeprazole.In vitro studies demonstrate that the solubility of GDC-0941 decreases with increasing pH, suggesting that changes in the pH of the upper GI tract may have effects on GDC-0941 solubility and hence on the rate and extent of its oral absorption and exposure. These data will be used to either modify dosing instructions regarding the use of a PPI while taking GDC-0941 and/or to provide specific dose administration instructions with respect to the timing of the use of a PPI with GDC-0941. Patients will self-administer rabeprazole 20 mg daily on Days 4 through 7. Rabeprazole will be co-administered with GDC-0941 on Day 8. Serial blood samples for pharmacokinetic (PK) assays were obtained on Days 1 and 8.

### GDC-0941 Formulations

Capsules of GDC-0941 are packaged in high-density polyethylene (HDPE) bottles and closed with child-resistant caps. The bottles are induction-sealed and labeled for clinical use. GDC-0941 is also manufactured as a film-coated, immediate-release tablet. The tablets are manufactured by a dry granulation process and are compressed into different size/weight tablets intended for PO administration. The formulated drug product will be provided as film-coated tablets in two strengths (20 and 100 mg), which are differentiated by size, shape, and weight of tablets. Tablets are packaged in HDPE bottles with desiccants and closed with a child-resistant cap. The bottles are induction sealed and labeled for clinical use.

### Rabeprazole

Rabeprazole sodium 20 mg (ACIPHEX^{®}) is available for oral administration as delayed-release, enteric-coated tablets.

### Dosage, Administration, and Storage

Dose escalations of approximately 50%, followed by dose escalations of approximately 33%, will be employed in subsequent cohorts.

In Stage 2 of the study, patients participating in the PPI-effect assessment will be administered the tablet formulation of GDC-0941. Based on data from the healthy volunteer study, the extent of GDC-0941 tablet exposure was slightly decreased relative to the capsule; however, this slight decrease is not considered clinically significant and does not present a risk to patients of higher or significantly lower exposures than they would receive with the capsule formulation. Other patients in Stage 2 may also receive the tablet formulation depending on capsule or tablet availability.

At each study visit, after establishing patient eligibility for continued administration of GDC-0941, a sufficient number of capsules or tablets should be dispensed to the patient to last only until the next visit or, at the investigator's discretion, through the dosing cycle.

Patients will be instructed as to the number and strength of capsules or tablets to take, according to their assigned dose level and schedule. To minimize the number of capsules or tablets administered, doses will be rounded so that a combination of no more than two different strengths will be required.

For QD dosing, patients should be instructed to take their dose at least 1 hour prior to their first meal of the day and at around the same time (no earlier than 1 hour and no later than 4 hours after the scheduled time; this dose timing should be observed as frequently as possible) each day that they take GDC-0941. On clinic days, dosing may be delayed for required protocol-specific procedures.

For the PPI-effect assessment in Stage 2, patients will receive a single dose of rabeprazole 20 mg (optimally in the morning; no dietary restrictions) on Days 4, 5, 6, and 7 of Cycle 1. The dose may be taken at home and patients will be asked to record the time and date that they take the rabeprazole in a medication diary. Patients will be instructed to return to the clinic on Day 8 prior to the first meal of the day and avoid consuming anything but water for 1 hour before dosing. A single dose of rabeprazole 20 mg will be administered, followed by the GDC-0941 dose with at least 3-4 ounces of water. Dosing of both medications should be completed within 5 minutes. Patients may have only water, as needed, for 1 hour after dosing.

### Example 3 pH Solubility profile for GDC-0941

The solubility of GDC-0941 was measured in different pH media. The solubility test was conducted using the shake flask method. The solubility of GDC-0941 was tested in 50mM USP buffer at pH values ranging from pH 1.0 to 7.5. Each sample was prepared at a volume of approximately 2 mL, with excess solid in each sample. All solubility samples were constantly mixing for 48 hours at 37 °C. Samples were then filtered and concentrations were determined by HPLC under reversed-phase conditions using a C18 column, and elution with a phosphate buffer (pH 6.5), acetonitrile gradient. The injection volume was 5 µL, with a flow rate of 1.2 mL/min and detection at 230 nm. The solubility of GDC-0941 ranged from approximately 0.8 mg/mL at pH 1.0 to <1 µg/mL at pH 7.5. The pH solubility profile for GDC-0941 is shown in Figure 2.

### Example 4 Dissolution of GDC-0941 with betaine-HCl

Dissolution of GDC-0941 85 mg tablets was conducted in 2mM sodium acetate buffer (900 mL, pH 5.0) containing various amounts of betaine HCl (100mg to 3000mg) as well as a control in which no betaine HCl was added. Experiments were performed at 37 °C using a USP Apparatus II dissolution unit with paddle rotation at 75 RPM. The concentration of GDC-0941 released at each time point was determined using on-line UV-Vis spectrophotometer with detection at 319 nm. Figure 3 shows the resulting dissolution profiles. Addition of the betaine HCl resulted in decrease in media pH and a corresponding improvement in tablet dissolution rate. Betaine HCl, also known as trimethyl glycine hydrochloride, has the formula (CH₃)₃N⁺CH₂CO₂H Cl⁻.

### Comparative Example 5 Oral exposure of dasatinib in dogs treated with famotidine or pentagastrin

Dasatinib pharmacokinetics was evaluated in dogs with famotidine-induced hypochlorhydria (Figure 5A). Dasatinib was administered as a 50 mg SPRYCEL® (Bristol-Myers Squibb) tablet. Famotidine 40 mg tablets were administered 3 hrs before dasatinib. Pentagastrin injection was administered intramuscularly 30 min prior before dasatinib administration. Two betaine HCl 750 mg tablets administered at the following times: 1 orally 20 min and 5 min prior to dasatinib administration.

Five groups of naive, male beagle dogs (n = 4 per group) each received a different pretreatment prior to dasatinib treatment.
Group 1: no pretreatment (control)
Group 2: famotidine 40 mg tablet orally, 3 hours prior to dasatinib
Group 3: pentagastrin by intramuscular injection (6 mg/kg) 30 min before dasatinib administration
Groups 4 and 5: Two betaine HCl 750 mg tablets were administered to the two groups as follows: one betaine HCl tablet orally 5 min before dasatinib and the other 20 min before dasatinib
Group 5: famotidine 40 mg tablet orally, 3 hours prior to dasatinib.

### Example 6 Bioavailability of GDC-0941 in dogs with famotidine-induced achlorhydria

Five groups of naive, male beagle dogs (n = 4 per group) each received a different pretreatment prior to GDC-0941 treatment (Figure 4).
Group 1: no pretreatment (control)
Group 2: famotidine 40 mg tablet orally, 3 hours prior to GDC-0941
Group 3: pentagastrin by intramuscular injection (6 mg/kg) 30 min before GDC-0941 administration
Groups 4 and 5: One betaine HCl 750 mg tablet were administered to the two groups as follows: one betaine HCl tablet orally 5 min before GDC-0941
Group 5: famotidine 40 mg tablet orally, 3 hours prior to GDC-0941.

Results strongly suggest that GDC-0941 is poorly absorbed when pH is increased by famotidine. Gastric acidification with betaine HCl (acid supplementation) performs nearly as well as pentagastrin (physiologic stimulation of HCl secretion) in treated dogs and betaine HCl improves GDC-0941 exposure in the presence of famotidine when compared to the control. Inter-animal PK variability is lower at maximal acidic gastric pH (pentagastrin and betaine HCl). The order of mean GDC-0941 exposure (AUC and Cmax) from high to low are: Group 3 (pentagastrin)> Group 4 (betaine HCl)> Group 5 (famotidine + betaine HCl)> Group 1 (Control) >> Group 2 (famotidine + GDC-0941).

### Comparative Example 7 Effects of betaine HCl on rabeprazole-induced hypochlorhydria on dasatinib pharmacokinetics in healthy volunteers

Dasatinib pharmacokinetics was evaluated in healthy volunteer subjects with rabeprazole-induced hypochlorhydria by dosing 20 mg oral rabeprazole twice daily for 4 days. On the morning (a.m.) of study day 5, subjects received an additional 20 mg oral rabeprazole, and the Heidelberg Capsule pH diagnostic system (HC) was used for continuous gastric pH monitoring. When gastric pH remained >4 for at least 15 min, a 1500 mg dose of betaine HCl was given orally, and gastric pH was monitored for 2 h. During part 2 of this study a 3-period crossover study, five of ten subjects each received: (A) 100 mg dasatinib (Control); (B) 100 mg dasatinib after rabeprazole pre-treatment; (C) 100 mg dasatinib, 1500 mg betaine HCl, after rabeprazole pre-treatment. For treatments B + C, 20 mg rabeprazole twice daily was given for 3 days prior to and on the a.m. of each study day. Gastric pH was monitored and DAS plasma concentrations were measured over 22 h (see study schematic below) with dasatinib concentration versus time results shown in Figure 8A and exposure (AUC) in Figure 8B.

| Treatment Group | Day 1 | Day 2 | Day 3 | Day 4 |
|---|---|---|---|---|
| A | --- | --- | --- | Dasatinib 100 mg |
| B | Rabeprazole 20 mg | Rabeprazole 20 mg | Rabeprazole 20 mg | Rabeprazole 20 mg |
| | | | | Dasatinib 100 mg |
| C | Rabeprazole 20 mg | Rabeprazole 20 mg | Rabeprazole 20 mg | Rabeprazole 20 mg |
| | | | | Betaine HCl 1500 mg |
| | | | | Dasatinib 100 mg |

## Claims

1. A re-acidification compound and GDC-0941 having the formula: 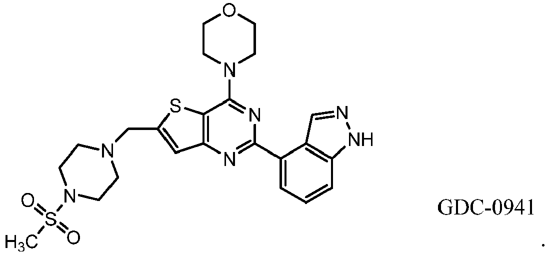 for use in a method of treating a hyperproliferative disorder, the method comprising administering a re-acidification compound and GDC-0941 to a patient receiving a gastric acid-reducing therapeutic selected from a proton-pump inhibitor, an H2-receptor antagonist, and an antacid, wherein the re-acidification compound is selected from betaine hydrochloride and glutamic acid hydrochloride.

2. The re-acidification compound and GDC-0941 for use in the method of treatment of claim 1, wherein the proton-pump inhibitor is selected from omeprazole, lansoprazole, rabeprazole, pantoprazole, and esomeprazole.

3. The re-acidification compound and GDC-0941 for use in the method of treatment of any one of the preceding claims, further comprising determining the gastrin level or the GDC-0941 concentration of a sample from the patient.

4. The re-acidification compound and GDC-0941 for use in the method of treatment of any one of the preceding claims, wherein the patient has a hyperproliferative disorder.

5. The re-acidification compound and GDC-0941 for use in the method of treatment of any one of the preceding claims, wherein the hyperproliferative disorder is a solid tumor or a haematological malignancy.

6. The re-acidification compound and GDC-0941 for use in the method of treatment of any one of the preceding claims, wherein the bioavailability of GDC-0941 in the patient is higher than when GDC-0941 and the gastric acid-reducing therapeutic are administered without the re-acidification compound.

7. The re-acidification compound and GDC-0941 for use in the method of treatment of any one of the preceding claims, wherein the pH of the gastric juices in the patient is lower than when GDC-0941 and the gastric acid-reducing therapeutic is administered without the re-acidification compound.

8. The re-acidification compound and GDC-0941 for use in the method of treatment of any one of the preceding claims, wherein GDC-0941 and the re-acidification compound is formulated in an oral dosage form.

9. The re-acidification compound and GDC-0941 for use in the method of treatment of any one of the preceding claims, wherein the method comprises coadministering the re-acidification compound and GDC-0941 to the patient.

## Patentansprüche

1. Wiederansäuerungsverbindung und GDC-0841 der Formel: 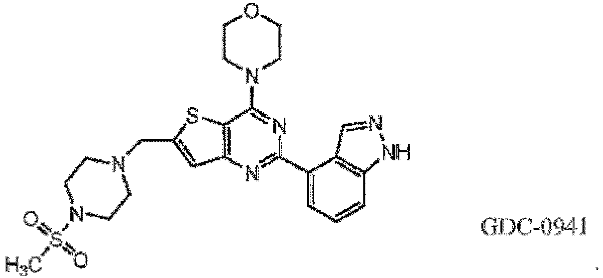 zur Verwendung in einem Verfahren zur Behandlung einer Hyperproliferationsstörung, wobei das Verfahren das Verabreichen einer Wiederansäuerungsverbindung und GDC-0941 an einen Patienten umfasst, der ein Magensäure reduzierendes Therapeutikum, ausgewählt aus einem Protonenpumpenhemmer, einem H2-Rezeptor-Antagonisten und einem Antazidum erhält, wobei die Wiederansäuerungsverbindung aus Betainhydrochlorid und Glutaminsäurehydrochlorid ausgewählt ist.

2. Wiederansäuerungsverbindung und GDC-0841 zur Verwendung in einem Behandlungsverfahren nach Anspruch 1, wobei der Protonenpumpenhemmer aus Omeprazol, Lansoprazol, Rabeprazol, Pantoprazol und Esomeprazol ausgewählt ist.

3. Wiederansäuerungsverbindung und GDC-0841 zur Verwendung in einem Behandlungsverfahren nach einem der vorangegangenen Ansprüche, das weiters die Ermittlung des Gastrin-Spiegels oder der GDC-0941-Konzentration einer Probe aus dem Patienten umfasst.

4. Wiederansäuerungsverbindung und GDC-0841 zur Verwendung in einem Behandlungsverfahren nach einem der vorangegangenen Ansprüche, wobei der Patient an einer Hyperproliferationsstörung leidet.

5. Wiederansäuerungsverbindung und GDC-0841 zur Verwendung in einem Behandlungsverfahren nach einem der vorangegangenen Ansprüche, wobei die Hyperproliferationsstörung ein solider Tumor oder ein hämatologisches Malignom ist.

6. Wiederansäuerungsverbindung und GDC-0841 zur Verwendung in einem Behandlungsverfahren nach einem der vorangegangenen Ansprüche, wobei die Bioverfügbarkeit von GDC-0941 in dem Patienten höher ist, als bei Verabreichung von GDC-0941 und dem Magensäure reduzierenden Therapeutikum ohne Wiederansäuerungsverbindung.

7. Wiederansäuerungsverbindung und GDC-0841 zur Verwendung in einem Behandlungsverfahren nach einem der vorangegangenen Ansprüche, wobei der pH der Magensäfte des Patienten niedriger ist als bei Verabreichung von GDC-0941 und dem Magensäure reduzierenden Therapeutikum ohne Wiederansäuerungsverbindung.

8. Wiederansäuerungsverbindung und GDC-0841 zur Verwendung in einem Behandlungsverfahren nach einem der vorangegangenen Ansprüche, wobei GDC-0941 und die Wiederansäuerungsverbindung in einer oralen Dosierungsform formuliert sind.

9. Wiederansäuerungsverbindung und GDC-0841 zur Verwendung in einem Behandlungsverfahren nach einem der vorangegangenen Ansprüche, wobei das Verfahren die gemeinsame Verabreichung der Wiederansäuerungsverbindung und von GDC-0941 an den Patienten umfasst.

## Revendications

1. Composé de ré-acidification et GDC-0941 répondant à la formule: 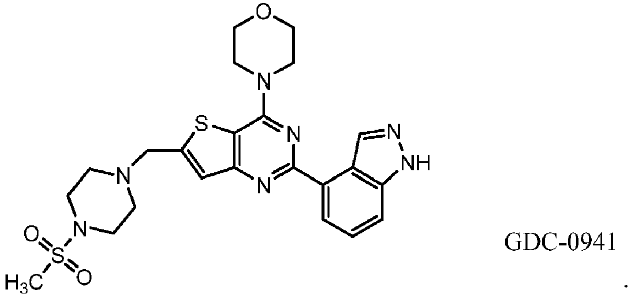 destinés à être utilisés dans une méthode de traitement d'un trouble hyperprolifératif, la méthode comprenant l'administration d'un composé de ré-acidification et GDC-0941 à un patient sous traitement thérapeutique visant à réduire l'acide gastrique choisi parmi un inhibiteur de pompe à protons, un antagoniste du récepteur H2, et un antacide, le composé de ré-acidification étant choisi parmi le chlorhydrate de bétaïne et le chlorhydrate d'acide glutamique.

2. Composé de ré-acidification et GDC-0941 destinés à être utilisés dans la méthode de traitement selon la revendication 1, l'inhibiteur de pompe à protons étant choisi parmi l'oméprazole, le lansoprazole, le rabéprazole, le pantoprazole, et l'ésoméprazole.

3. Composé de ré-acidification et GDC-0941 destinés à être utilisés dans la méthode de traitement selon l'une quelconque des revendications précédentes, comprenant en outre la détermination du taux de gastrine ou de la concentration de GDC-0941 dans un échantillon provenant du patient.

4. Composé de ré-acidification et GDC-0941 destinés à être utilisés dans la méthode de traitement selon l'une quelconque des revendications précédentes, le patient souffrant d'un trouble hyperprolifératif.

5. Composé de ré-acidification et GDC-0941 destinés à être utilisés dans la méthode de traitement selon l'une quelconque des revendications précédentes, le trouble hyperprolifératif étant une tumeur solide ou une malignité hématologique.

6. Composé de ré-acidification et GDC-0941 destinés à être utilisés dans la méthode de traitement selon l'une quelconque des revendications précédentes, la bio-disponibilité du GDC-0941 chez le patient étant supérieure que dans le cas où le GDC-0941 et l'agent thérapeutique réduisant l'acide gastrique sont administrés sans le composé de ré-acidification.

7. Composé de ré-acidification et GDC-0941 destinés à être utilisés dans la méthode de traitement selon l'une quelconque des revendications précédentes, le pH des sucs gastriques chez le patient étant inférieur que dans le cas où le GDC-0941 et l'agent thérapeutique réduisant l'acide gastrique sont administrés sans le composé de ré-acidification.

8. Composé de ré-acidification et GDC-0941 destinés à être utilisés dans la méthode de traitement selon l'une quelconque des revendications précédentes, le GDC-0941 et le composé de ré-acidification étant formulés dans une forme pharmaceutique à usage oral.

9. Composé de ré-acidification et GDC-0941 destinés à être utilisés dans la méthode de traitement selon l'une quelconque des revendications précédentes, la méthode comprenant la co-administration du composé de ré-acidification et du GDC-0941 au patient.
